# FASCICULE DE BREVET EUROPEEN

(11) **EP 1 479 374 B1**
(45) Date de publication et mention de la délivrance du brevet: **29.03.2017**
(21) Numéro de dépôt: 04290875.6
(22) Date de dépôt: 01.04.2004
(51) Int. Cl.: A61K 8/49, A61K 8/67, A61Q 19/00

(54) **Procédé de traitement cosmétique des rougeurs**
Zusammensetzung und kosmetisches Behandlungsverfahren für Hautrötungen
Process for the cosmetic treatment of cutaneous erythema or skin redness

(30) Priorité: 22.05.2003 FR 0306159
(43) Date de publication de la demande: 24.11.2004
(73) Titulaire: L'Oréal, 75008 Paris (FR)
(72) Inventeur: Marion, Catherine, 92160 Antony (FR); Sore, Gabrielle, 75012 Paris (FR)
(74) Mandataire: Brohmi, Karim

(56) Documents cités:
- WO-A-00/69408
- WO-A-03/039418
- WO-A1-96/19182
- FR-A- 2 831 440
- GB-A- 2 221 391
- GB-A- 2 335 142
- US-A- 5 523 090
- US-A- 5 840 681

## Description

La présente invention se rapporte à un procédé de traitement cosmétique des rougeurs cutanées, comprenant l'application topique sur la peau d'une composition renfermant de la caféine dans un milieu physiologiquement acceptable.

La rosacée est une dermatose bénigne chronique qui touche essentiellement le visage de sujets à peau fine et claire, entre 30 et 60 ans, plus particulièrement des femmes. Elle se caractérise par un érythème facial persistant, des bouffées (flushs) fréquentes et des télangiectasies. Bien que des papules et pustules puissent survenir par intermittence, la rosacée ne doit pas être confondue avec l'acné, dans la mesure où les comédons et les kystes qui caractérisent l'acné ne sont pas des symptômes de la rosacée.

Précisément, on distingue quatre stades dans l'évolution de la rosacée :
- le stade des « flushs » ou bouffées vasomotrices du visage et du décolleté, déclenchées par des changements de température, des efforts physiques, des émotions, des expositions solaires ou lors de l'ingestion de certains aliments ou boissons. Ce phénomène est dû à un retard de la vidange des plexus veineux ;
- le stade de la couperose ou de l'érythro-couperose qui correspond à un état érythémateux permanent du visage associé à des télangiectasies. L'érythrose a une couleur variable qui peut aller du rose clair au rouge soutenu voire violacé. Ce stade peut parfois s'accompagner d'un oedème dur permanent ;
- le stade inflammatoire avec l'épisode de pustules et de papules inflammatoires sur un fond érythémato-télangiectasique, phase d'état caractéristique de la rosacée ; et
- le stade du Rhinophyma qui est essentiellement masculin et se traduit par le gonflement constant de certaines zones du visage. Le faciès est rubicond et le nez rouge et gros, bosselé, associé à une hyperplasie sébacée et à un remaniement fibreux du tissu conjonctif.

Différents traitements efficaces ont été proposé, qui peuvent non seulement freiner la progression de la rosacée, mais aussi remédier aux symptômes décrits ci-dessus. Il peut s'agir de traitements cosmétiques, dermatologiques ou physiques.

Ainsi, il est connu d'utiliser des produits anti-couperosiques, à effet vasculotrope, pour traiter la couperose légère à modérée caractéristique des deux premiers stades de la rosacée. Au troisième stade, les lésions inflammatoires peuvent être traitées par un gel ou une crème antibiotiques à base de métronidazole ou d'acide azélaïque, par exemple. En cas d'atteinte plus sévère, une antibiothérapie par voie orale peut être associée au traitement topique. L'utilisation de tétracyclines est recommandée à ce stade. Au quatrième stade de la rosacée, seul le recours à des méthodes physiques telles que le laser est possible car aucun médicament n'est efficace.

Or, ces traitements, et notamment les produits cosmétiques appliqués topiquement sur la peau couperosique, ont l'inconvénient d'être relativement irritants et donc mal tolérés par les sujets qui ont généralement une peau très sensible. Les traitements les moins irritants sont par ailleurs les moins efficaces.

On comprend donc l'intérêt qu'il y a à agir dès les premiers stades de la rosacée pour éviter son aggravation, en ayant recours à des traitements cosmétiques non agressifs pour la peau qui pourront également être utilisés au troisième stade de la rosacée, en accompagnement de l'antibiothérapie.

Or, la Demanderesse a découvert, de manière surprenante et inattendue, que la caféine permettait d'atténuer l'érythro-couperose qui est le stade précoce de la rosacée.

Cette découverte est d'autant plus surprenante que la littérature mentionne le rôle aggravant de la consommation de café sur la rosacée (WILKIN J. K., Oral thermal-induced flushing in erythematotelangiectatic rosacea, Journal of Investigative Dermatology, Janvier 1981, 76(1) :15-8) et pourrait venir conforter l'hypothèse selon laquelle cette condition serait davantage déclenchée par la chaleur de la boisson que par sa teneur en caféine (Diagnosing an inflamed situation (Rosacea analysis), Chemist & Druggist, 26 Mai 2001, p. 26 ; Restaurant flushing syndrome : make sure you identify its cause, Dermatology Times, Juillet 1993, p. 1).

Si ces deux dernières publications remettent en cause le rôle de la caféine sur la rosacée, elles ne constatent toutefois pas d'amélioration de la rosacée lors de la prise de caféine et ne permettent pas a *fortiori* de laisser penser que la caféine, appliquée topiquement sur la peau, peut avoir cet effet.

Le brevet US-6,352,698 décrit une composition destinée au traitement des peaux sensibles, comprenant un complexe hypoallergénique qui peut être constitué d'une association de lactoferrine, de driéline, de panthénol et d'extrait de thé vert renfermant de la caféine. Il est indiqué que ce complexe a la propriété d'abaisser le seuil de réactivité de la peau et de diminuer l'amplitude des réactions d'intolérance ou immunoallergiques. Ce complexe agirait en diminuant la synthèse ou l'expression de neuromédiateurs. La composition le contenant pourrait être utilisée pour préparer un médicament immunomodulateur utilisable notamment dans le traitement de la rosacée.

Toutefois, à ce jour, le lien entre immunodéficience et rosacée n'a jamais été démontré. En outre, les symptômes des peaux sensibles que le complexe décrit dans ce brevet vise à atténuer ne sont pas précisément décrits et on comprend que ce complexe n'agit pas nécessairement sur la rougeur cutanée mais peut en variante diminuer les picotements, les douleurs ou encore le prurit classiquement associés aux peaux sensibles.

Ainsi, il n'était pas évident que la caféine, qui est l'un des constituants minoritaires des extraits de feuilles de thé vert, pouvait avoir un effet préventif et curatif notable sur les rougeurs cutanées, en particulier celles liées aux stades précoces de la rosacée.

Par ailleurs, on connaissait de FR-2 831 440 l'effet apaisant des extraits de Cola nitida. Toutefois, la caféine est associée dans les noix de Cola à d'autres principes actifs que sont notamment les tannins et la théobromine. Par conséquent, là encore, il n'était pas évident que la caféine puisse elle-même avoir un effet sur les rougeurs cutanées.

Enfin, on connaissait de WO 03/039418 l'effet anti-irritant de la caféine. Il n'était toutefois pas précisé sur quelle composante de l'irritation ce composé agissait.

Or, la Demanderesse a montré que la caféine agissait efficacement sur les rougeurs.

La présente invention a donc pour objet un procédé de traitement cosmétique des rougeurs cutanées, comprenant l'application topique sur la peau d'une composition comprenant de la caféine dans un milieu physiologiquement acceptable.

Le procédé selon l'invention est destiné à prévenir ou atténuer les rougeurs cutanées d'origines diverses, en particulier celles associées aux stades précoces de la rosacée. Il peut en variante être mis en oeuvre sur des peaux irritables ou irritées, que ce soit par des traitements chimiques (peelings chimiques), physiques (laser) ou médicamenteux (acide rétinoïque, hydroxyacides). Il peut en variante être utile dans la prévention ou le traitement des rougeurs d'origine inflammatoire, en particulier des folliculites, telles que celles subséquentes au rasage.

La composition selon l'invention est donc avantageusement appliquée sur des personnes présentant au moins l'un des symptômes suivants : une rosacée, une peau irritée ou irritable ou une folliculite. Il est par exemple mis en oeuvre subséquemment à un peeling chimique ou physique.

La composition selon l'invention est de préférence appliquée sur le visage.

Elle contient un milieu physiologiquement acceptable et une quantité efficace de caféine, par exemple une quantité comprise entre 0,05% et 3% en poids, de préférence entre 0,1 et 1% en poids, par rapport au poids total de la composition.

Par milieu physiologiquement acceptable, on comprend un milieu compatible avec la peau et éventuellement avec les muqueuses, les ongles, le cuir chevelu et/ou les cheveux.

La composition selon l'invention peut avoir la forme notamment d'une solution aqueuse ou d'une dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel, ou encore de microcapsules ou microparticules, ou de dispersions vésiculaires de type ionique et/ou non ionique. Elle peut éventuellement être appliquée sur la peau sous forme d'aérosol. Elle peut également se présenter sous forme solide, et par exemple sous forme de stick. Ces compositions sont préparées selon les méthodes usuelles.

Cette composition peut être utilisée comme produit de soin, comme produit de nettoyage ou comme produit de maquillage de la peau.

Lorsque la composition utilisable selon l'invention est une émulsion, la proportion de la phase grasse peut aller de 5% à 80% en poids, et de préférence de 5% à 50% en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3% à 30% en poids, et de préférence de 0,5 à 20% en poids par rapport au poids total de la composition. L'émulsion peut, en outre, contenir des vésicules lipidiques.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).

Comme émulsionnants utilisables dans l'invention, on peut citer par exemple le monostéarate de glycérol, le polysorbate 60 et les stéarates de polyéthylène glycol (20 OE, 40 OE, 100 OE).

De façon connue, la composition de l'invention peut contenir également les adjuvants habituels dans les domaines cosmétiques et dermatologique, tels que les gélifiants hydrophiles ou lipophiles, les actifs hydrophiles ou lipophiles, les conservateurs, les antioxydants, les solvants, les parfums, les charges, les filtres, les pigments, les agents chélateurs, et les matières colorantes. Les quantités de ces différents adjuvants sont celles classiquement utilisées dans les domaines considérés, et par exemple de 0,01% à 20 % du poids total de la composition. Ces adjuvants, selon leur nature, peuvent être introduits dans la phase grasse, dans la phase aqueuse, dans les vésicules lipidiques et/ou dans les nanoparticules. Ils seront choisis de manière à ne pas nuire aux propriétés recherchées selon l'invention.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe.

Comme actifs, on préfère que la composition selon l'invention renferme en outre au moins un composé choisi parmi : l'acide ascorbique et ses dérivés tels que le glucoside d'ascorbyle, l'ascorbyl phosphate de magnésium et leur mélange ; la niacinamide ; le tocophérol et ses dérivés tels que ses esters, en particulier l'acétate de tocophérol ; les galactolipides, extraits notamment d'avoine.

D'autres caractéristiques et avantages de l'invention ressortiront mieux des exemples qui suivent, donnés à titre illustratif et non limitatif. Dans ce qui suit, les proportions sont données en pourcentage pondéral, sauf indication contraire.

### EXEMPLES

### Exemple 1 : Etude in vivo

### a) Protocole

Deux formules A et B contenant de la caféine ont été appliquées chacune sur un panel, respectivement de 50 et 62 femmes présentant une rosacée de stade 2 (érythème permanent et/ou télangiectasies), pendant deux mois, à raison de deux fois par jour. Une évaluation clinique dermatologique, et des mesures par laser Doppler et chromamétrie ont été effectuées au début du traitement et au bout de huit semaines.

Ces formules avaient la composition suivante :

| | **Formule A** | **Formule B** |
|---|---|---|
| Neutralisant | 0,18% | 0,17% |
| Niacinamide | 4% | 4% |
| Colorants | qs | qs |
| Tensioactif non ionique | 3% | 3% |
| Conservateurs | qs | qs |
| Alcool | 5% | 5% |
| Acétate de tocophérol | 0,2% | 0,2% |
| Citrate de sodium | 0,3% | 0,3% |
| **Caféine** | **0,3%** | -- |
| **Rutinyl disulfate de sodium** | **--** | **1%** |
| **Vitamine K (phytonadione)** | **--** | **0,5%** |
| Epaississants | 0,4% | 0,4% |
| Glycérine | 5% | 5% |
| Isononanoate d'isononyle | 10% | 10% |
| Alcool cétylique | 1,5% | 1,5% |
| Huile de silicone | 5% | 5% |
| Glucoside d'ascorbyle | 1,5 % | 1,5% |
| Charges | 8% | 8% |
| Glycolipides | 0,25% | 0,25% |
| Acide citrique | 0,05% | 0,05% |
| Eau | qsp 100% | qsp 100% |

### b) Résultats

Au bout de huit semaines de traitement avec la formule B (exemple comparatif), on n'a observé aucune variation significative du paramètre "a" (couleur rouge) en chromamétrie et une légère diminution du paramètre "L", qui traduit un assombrissement de la peau. En revanche, la diminution du débit sanguin au niveau du visage, mesurée par laser Doppler, est significative.

Par comparaison, au bout de huit semaines de traitement avec la formule A (exemple selon l'invention), on a observé une diminution significative du paramètre "a" mesuré par chromamétrie, et donc de la rougeur du visage, ainsi qu'une augmentation significative du paramètre "L" qui traduit un éclaircissement significatif du teint. La réduction du débit sanguin au niveau du visage est très significative.

### c) Conclusion

Les résultats ci-dessus montrent que la formule A selon l'invention atténue plus efficacement les rougeurs caractéristiques des stades précoces de la rosacée que la formule B qui contient des actifs vasculoprotecteurs - supposés efficaces contre la rosacée d'après la littérature-.

### Exemple 2 : compositions cosmétiques

### 2.1 - Emulsion H/E

| | |
|---|---|
| Caféine | 1 % |
| Isononanoate d'isononyle | 10 % |
| Talc | 8 % |
| Gomme de xanthane | 0,2 % |
| Alcool | 5 % |
| Glycérine | 5 % |
| Mélange de stéarate de glycéryle | |
| et de stéarate oxyéthyléné (100 OE) | 3 % |
| Alcool cétylique | 1,5 % |
| Copolymère acrylique (Pemulen TR2) | 0,3 % |
| Hydroxyde de sodium | 0,2 % |
| Conservateurs | 1 % |
| Eau | qsp 100 % |

### 2.2 - Emulsion E/silicones

| | |
|---|---|
| Caféine | 0,1 % |
| Huile d'abricot | 6 % |
| Cyclopentasiloxane et polydiméthylsiloxane | |
| oxyéthyléné (18 OE) et oxypropyléné (18 OP) | 15 % |
| Glycérine | 23 % |
| Charges | 0,5 % |
| Conservateurs | 1 % |
| Hydroxyde de sodium | 1,8 % |
| Eau | qsp 100 % |

## Revendications

1. Composition comprenant de la caféine dans un milieu physiologiquement acceptable, destinée à être utilisée pour prévenir ou atténuer les rougeurs cutanées des folliculites ou les rougeurs cutanées associées aux stades précoces de la rosacée, **caractérisée en ce qu'**elle est mise en oeuvre par application topique sur la peau du visage.

2. Composition pour son utilisation selon la revendication 1, **caractérisée en ce qu'**elle renferme de 0,1 à 1% en poids de caféine, par rapport à son poids total.

3. Composition pour son utilisation selon l'une quelconque des revendications 1 à 2, **caractérisée en ce qu'**elle renferme en outre au moins un composé choisi parmi :
l'acide ascorbique et ses dérivés ; la niacinamide ; et le tocophérol et ses dérivés.

4. Composition pour son utilisation selon la revendication 3, **caractérisée en ce que** les dérivés d'acide ascorbique sont choisis parmi : le glucoside d'ascorbyle, l'ascorbyl phosphate de magnésium et leur mélange.

5. Composition pour son utilisation selon la revendication 3, **caractérisée en ce que** les dérivés de tocophérol sont choisis parmi les esters de tocophérol, en particulier l'acétate de tocophérol.

## Patentansprüche

1. Zusammensetzung, die Koffein in einem physiologisch annehmbaren Medium umfasst, für die Verwendung zur Vorbeugung oder Abschwächung der Hautrötungen von Follikulitiden oder der durch Frühstadien von Rosazea bedingten Hautrötungen, dadurch gekenntzeichnet, dass sie mittels topischer Applikation auf die Gesichtshaut eingesetzt wird.

2. Zusammensetzung für die Verwendung nach Anspruch 1, **dadurch gekennzeichnet, dass** sie 0,1 bis 1 Gew.-% Koffein, bezogen auf ihr Gesamtgewicht, umfasst.

3. Zusammensetzung für die Verwendung nach einem der Ansprüche 1 bis 2, **dadurch gekennzeichnet, dass** sie außerdem mindestens eine Verbindung umfasst, die aus den folgenden ausgewählt ist: Ascorbinsäure und deren Derivaten, Niacinamid sowie Tocopherol und dessen Derivaten.

4. Zusammensetzung für die Verwendung nach Anspruch 3, **dadurch gekennzeichnet, dass** die Ascorbinsäurederivate aus den folgenden ausgewählt sind: Ascorbylglucosid, Magnesiumascorbylphosphat und deren Gemisch.

5. Zusammensetzung für die Verwendung nach Anspruch 3, **dadurch gekennzeichnet, dass** die Tocopherolderivate aus Tocopherolestern, insbesondere Tocopherolacetat, ausgewählt sind.

## Claims

1. Composition comprising caffeine in a physiologically acceptable medium, intended to be used to prevent or alleviate skin redness from folliculitis or skin redness associated with the early stages of rosacea, **characterized in that** it is employed by topical application to the skin of the face.

2. Composition for use thereof according to Claim 1, **characterized in that** it contains from 0.1 to 1% by weight of caffeine relative to the total weight thereof.

3. Composition for use thereof according to either one of Claims 1 and 2, **characterized in that** it also contains at least one compound chosen from: ascorbic acid and derivatives thereof, niacinamide, and tocopherol and derivatives thereof.

4. Composition for use thereof according to Claim 3, **characterized in that** the ascorbic acid derivatives are chosen from: ascorbyl glucoside, magnesium ascorbyl phosphate and the mixture thereof.

5. Composition for use thereof according to Claim 3, **characterized in that** the tocopherol derivatives are chosen from esters of tocopherol, in particular tocopherol acetate.
